Europäisches Patentamt

European Patent Office

Office européen des brevets

(19)

(11) Publication number: **0 119 176**
**A2**

(12) # EUROPEAN PATENT APPLICATION

(21) Application number: **84850032.8**

(22) Date of filing: **01.02.84**

(51) Int. Cl.³: **G 01 N 1/06**

(30) Priority: **11.02.83 SE 8300735**

(43) Date of publication of application:
**19.09.84 Bulletin 84/38**

(84) Designated Contracting States:
**DE FR GB**

(71) Applicant: **LKB-Produkter AB**
**Box 305**
**S-161 26 Bromma(SE)**

(72) Inventor: **Forsström, Bo**
**S:t Eriksplan 10**
**S-113 32 Stockholm(SE)**

(72) Inventor: **Hellquist, Henrik**
**Västantorpsvägen 17**
**S-597 00 Atvidaberg(SE)**

(74) Representative: **Johansson, Lars E.**
**LKB-Produkter AB Box 305**
**S-161 26 Bromma(SE)**

(54) **Method of cutting, in a microtome, a tissue sample, and sample taking needle for taking such a sample.**

(57) The invention relates on the one hand to a method of cutting, in a microtome, a tissue sample taken by means of a sample taking needle (6) cooled down to a low temperature, and, on the other hand, the sample taking needle (6) itself. According to the invention at least the sample taking end of the needle (6) is made of such a material that the tissue sample frozen to the needle (6) can be cut together with the needle (6) itself.

Fig. 2

TECHNICAL FIELD

The present invention relates to a method of cutting, in a microtome, a tissue sample taken by means of a sample taking needle cooled down to a low temperature, and a sample taking needle for taking such a tissue sample.

BACKGROUND ART

When carrying out quantitative and qualitative analysis of e.g. elements, chemical compounds and antibodies in living cells by e.g. electron microscopy or X-ray spectroscopy one wants to avoid cell changes from the moment of taking the samples until the analysis is carried out.

It is known to take samples from living organisms by means of cooled sample taking needles onto which the samples freeze inside the organism. Before these samples can be cut in the thin cuts in which they have to be present in e.g. electron microscopy analysis, the samples have to be removed from the sample taking needles.

The drawback of this method is that the cells can undergo disadvantageous changes.

DISCLOSURE OF INVENTION

The object of the present invention is therefore to bring about a cutting method and a sample taking needle that substantially eliminate the risk of cell changes.

This is obtained in that the method and the needle according to the invention have obtained the characterizing features defined in the claims.

BRIEF DESCRIPTION OF DRAWING

The invention will now be explained in detail with reference to the attached drawing on which

Fig. 1 illustrates the principle of taking tissue samples from living organs, and

Fig. 2 shows an embodiment of a sample taking needle in accordance with the invention, that can be used in the device shown in Fig. 1.

DETAILED DESCRIPTION

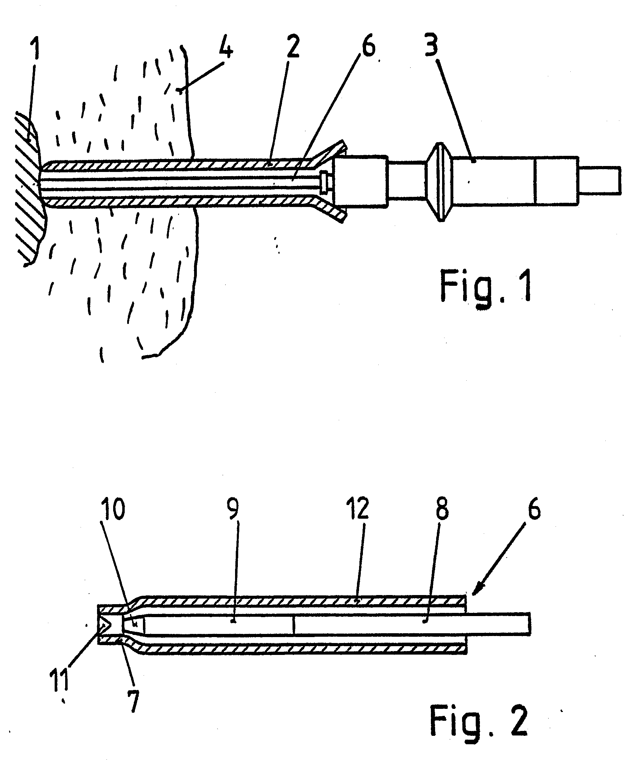

In Fig. 1 the reference character 1 denotes an organ, e.g. a kidney, from which a tissue sample is to be taken, and 4 denotes the surrounding tissue. When taking the sample a guide tube 2 is in a manner known per se inserted through the surrounding tissue into contact with the organ in question. The sample taking needle 6 which is carried by a holder 3 and which in a manner known per se can be pressed into the organ 1, is then inserted into the guide tube 2.

The sample taking needle according to the invention is, at the sample taking moment, cooled down to a temperature, of $-150^{\circ}$C at the most which results in that the tissue is immediately cooled down upon contact with the needle and freezes to the tip of the needle. In Fig. 2 an embodiment of the sample taking needle according to the invention is shown. The sample taking needle 6 shown comprises an outer insulating sleeve 12 within which two wires 8 and 9 are joined end to end. The free end of the sleeve 12 is adapted to act as a guide for the free end of the wire 9, which constitutes the sample taking end of the needle 6. In the embodiment shown the wire 9 is preferably made of a material having a high thermal conductivity and is at its free end provided on the one hand with a cone-shaped recess 11, and on t other hand with a ring-shaped groove 10. When the sample taking end is brought out of the sleeve 12 and comes into contact with the tissue, the tissue will immediately freeze to the contact surface. The ring-shaped groove 10 prevents the frozen tissue from getting loose from the needle 6 when it is removed from the sample taking area. Due to the low

temperature of the needle a shock freezing of the sample takes place. A prerequisite condition is however that the sample taking takes place during a very short period of time and that the heat transport in the needle is instantaneous. For analyzing the sample in an electron microscope the needle together with the tissue sample is, in accordance with the invention, cut in an ultramicrotome into thin slices having a thickness of about 100 nm. To make this possible the wire 9 is made of a material that can be cut in a microtome, e.g. copper. The purpose of the cone-shaped recess 11 at the free end of the wire 9 is to make it possible to locate the tissue to be analyzed after cutting. When the slices contain rings of the wire 9 the slices can be analyzed. The wire 8 which is joinedto the wire 9 is made of a material having a low thermal conductivity, e.g. stainless steel or some plastic material, suitable for the purpose. When cooling the needle the heat transport in the wire will, thus, be very slow from the atmosphere outside the cooling device to the wire having high thermal conductivity.

The sleeve 12 is made of a material having a low thermal conductivity, e.g. polytetrafluoroethylene. The material shall withstand sterilization at +120$^{O}$C and cooling down to -195$^{O}$C. In order to ensure shockfreezing upon taking of the sample at least some part of the sample taking end of the needle has to be free from insulating ice crystals. The sleeve 12 is therefore provided with a constriction 7 at the end that encloses the sample taking part of the needle. Thereby, this will be closely enclosed within the sleeve 12.

Claims          .     .       **0119176**

1. Method of cutting, in a microtome, a tissue
   sample taken by means of a sample taking needle (6)
   cooled down to a low temperature, characterized in
   that the tissue sample is cut together with the
   needle (6).

2. Sample taking needle for taking a tissue sample to
   be cut in a microtome, said needle being cooled down
   to a low temperature before the sample is taken in
   order for the sample to freeze to the sample taking
   end of the needle (6), characterized in that at least
   the sample taking end of the needle (6) is made of a
   material that can be cut together with the sample frozen
   to said end.

3. Needle according to claim 2, characterized in that the
   sample taking end has at least one peripheral recess (10)
   for retaining the tissue sample frozen to said end.

4. Needle according to claim 3, characterized in that the
   recess extends around the sample taking end.

5. Needle according to any of claims 2-4, characterized in
   that the sample taking end exhibits an axial recess (11).

6. Needle according to any of claims 2-5, characterized
   in that a heat insulating sleeve (12) is adapted to
   closely surround at least the sample taking end of the
   needle (6) in order to prevent the formation of insulating
   ice crystals on said end before the taking of the sample,
   said end being brought out of said sleeve (12) in connection
   with the taking of the sample.

Fig. 1

Fig. 2